# EUROPEAN PATENT APPLICATION

(11) **EP 1 715 061 A1**
(43) Date of publication of application: **25.10.2006**
(21) Application number: 05018191.6
(22) Date of filing: 22.08.2005
(51) Int. Cl.: C12Q 1/68

(54) **Multiplex SNP typing by bioluminometric assay coupled with terminator incorporation**

(30) Priority: 21.04.2005 JP 2005124077; 19.07.2005 JP 2005208698
(71) Applicant: HITACHI, LTD., Chiyoda-ku Tokyo 100-8280 (JP)
(72) Inventor: Kambara, Hideki c/o Hitachi, Ltd., 6-1, Marunouchi 1-chome Tokyo 100-8220 (JP); Zhou, Guohua c/o Hitachi, Ltd., 6-1, Marunouchi 1-chome Tokyo 100-8220 (JP); Kajiyama, Tomoharu c/o Hitachi, Ltd., 6-1, Marunouchi 1-chome Tokyo 100-8220 (JP)
(74) Representative: Strehl Schübel-Hopf & Partner

(57) **Abstract**

The present invention provides a method for analyzing a nucleotide sequence comprising the steps of: carrying out complementary strand synthesis by adding at least one of four kinds of ddNTP corresponding to nucleotides A, G, T, and C, or derivatives thereof to a reaction vessel containing a nucleic acid sample to extend one nucleotide at a target site; performing a bioluminescent reaction with the use of ATP formed from released pyrophosphate as a reaction substrate; and typing the target site by determining the presence or absence of the complementary strand synthesis based on a result of the bioluminescent reaction. The method of the present invention allows multiplex SNPs to be typed in one reaction vessel

## Description

### CLAIM OF PRIORITY

The present application claims priority from Japanese applications JP 2005-124077 filed on April 21, 2005 and JP 2005-208698 filed on July 19, 2005, the contents of which are hereby incorporated by reference into this application.

### FIELD OF THE INVENTION

The present invention relates to a method for analyzing a nucleotide sequence. More specifically, the present invention relates to a method for analyzing nucleotide mutations and variations such as SNPs (single nucleotide polymorphisms) and methylated cytosine and a method of gene diagnosis making use thereof.

### BACKGROUND OF THE INVENTION

After completion of the human genome analysis, an era of making full use of the genome information in various fields has come. Elucidation of human identity and drug sensitivity has also progressed at the genetic level, and its outcomes are going to be applied to diagnosis and treatment. For these purposes, it is considered that SNPs (single nucleotide polymorphisms) present in the genome sequence play a particularly important role. Thus SNP databases are being constructed and beginning to be utilized in various diagnoses.

There are numerous methods proposed for SNP analysis including a method of DNA sequencing, a method in which a fluorescently labeled nucleotide corresponding to a mutation is allowed to be incorporated into the mutation site and then examined by gel electrophoresis, a method in which a fluorescent probe designed to become active depending on the presence or absence of the mutation site is used, a method in which whether a probe is stably hybridized to a target or not is determined as is the case with DNA chip or the like, and a method of sequencing by stepwise complementary strand synthesis. Many of these methods belong to technologies developed for construction of SNP databases and often require an elaborate and expensive apparatus. Therefore, a method of SNP typing that is easy to use and low in running cost has been desired.

Methods for typing of DNA sequence are broadly divided into two methods in which one method makes use of fluorescence and the other makes use of bioluminescence. Since the fluorescence method requires a light source, the apparatus generally tends to become larger. On the other hand, the bioluminescence method using luciferase reaction (bioluminometric assay) is recently utilized in pyrosequencing for typing of DNA sequence, though the luciferase reaction has been conventionally used mainly in ATP determination and bacteriological examination (detecting ATP).

In pyrosequencing, pyrophosphate generated by DNA complementary strand synthesis is converted to ATP, which is used as a substrate for the luciferin-luciferase reaction to generate light for detection, thereby allowing sequencing. More specifically, nucleotide substrates are added to a reaction cell one by one, and reactions of complementary strand synthesis are carried out. When light is observed, it is regarded that a complementary strand synthesis took place, thereby making it possible to determine the kind of a nucleotide at the incorporated site from the added nucleotide substrate. After the reaction, an excess substrate is promptly degraded by an enzyme so that the substrate may hardly exist in the reaction cell when the next nucleotide substrate is added. Since the amount of luminescence is proportional to the amount of the complementary strand synthesis, a double amount of luminescence is observed when a double amount of the substrate is incorporated at a time.

In human genomic DNA, two alleles derived from mother and farther respectively are paired, and there are two cases in which the sequence at a specific site of a DNA sample has two different kinds of alleles (heterozygous sample) and identical alleles (homozygous sample). In the case of pyrosequencing, the signal intensity obtained from the heterozygous sample is a half of that from the homozygous sample, and this half signal sometimes persists for a while depending on the sample. Such a phenomenon is troublesome to acquire accurate information on sequences including SNPs.

In DNA complementary strand synthesis, the complementary strand is generally synthesized using four nucleotides, dATP, dCTP, dGTP, and dTTP. Since the structure of dATP is similar to that of ATP that is a substrate for the luminescent reaction, dATP also serves as a substrate for the luciferase reaction. In other words, when dATP is added to perform complementary strand synthesis, the luciferase reaction takes place, and therefore luminescence is observed even if complementary strand synthesis does not take place. On the other hand, a method in which the complementary strand synthesis is carried out using a dATP analog such as dATPαS or ddATP that does not serve as a substrate for the luminescent reaction has been developed (refer to WO 98/13523 (Published Japanese Translations of PCT International Publication for Patent Applications 2001-501092)), though there is a problem that its running cost becomes high because dATPαS is expensive.

Further, in the luciferase reaction with the use of ATP, pyrophosphate is formed as a reaction product in concurrence with luminescence emission, and the pyrophosphate is converted again to ATP to serve as a substrate for the luminescent reaction. That is, the luminescence persists over a prolonged period. To solve this problem, a method in which added dNTP and ATP are rapidly degraded by allowing a degrading enzyme, apyrase, to be present in the reaction solution so that luminescence (signal) may be generated every time when a nucleotide substrate is added has been developed (refer to WO 98/28440 (Published Japanese Translations of PCT International Publication for Patent Applications 2001-506864)). However, when the concentration of apyrase is high, the luminescent reaction is retarded, and when the concentration of apyrase is low, the reaction due to remaining dNTP takes place. Therefore, there are problems that its use is difficult and its cost is high.

In addition to these methods, a method in which four kinds of fluorescently labeled terminators are added to a reaction cell at a time to carry out a single nucleotide extension for complementary strand, thereby determining mutation in DNA, has been reported (refer to Science 1987, 238, 336-341). In this method, however, fluorescence due to incorporated fluorescently labeled terminators and fluorescence due to excess terminators are separated after the complementary strand extension, and therefore the product must be separated by gel electrophoresis before detection. Further, a method in which four kinds of terminators are added to the reaction cell at the same time to carry out one nucleotide extension reaction and then the product is analyzed by a mass spectrometer has also been reported (refer to Nucleic Acids Research, 2000, Vol. 28, No. 12), though this method lacks in general versatility because an expensive instrument is required.

### SUMMARY OF THE INVENTION

An object of the present invention is to provide an inexpensive and simple method for analyzing a nucleotide sequence in which the problems of conventional technologies are solved, particularly a method of typing capable of detecting multiplex nucleotide mutations and variations such as SNPs in one reaction vessel.

In order to solve the above problems, the present invention provides an inexpensive and simple method for analyzing a nucleotide sequence with high reproducibility by bioluminometric assay coupled with terminator incorporation based on the luciferin-luciferase reaction. In conventional pyrosequencing, an added nucleotide substrate had to be degraded by apyrase in order not to exert an influence upon a subsequent reaction, or an expensive reagent such as dATPαS had to be used to reduce background luminescence. These problems have been solved in the method of the present invention by using nucleotide analogs (ddNTP) with which a reaction of complementary strand synthesis can be terminated after addition of one nucleotide and further extension of complementary strand is not possible (because of this property, ddNTP is referred to as terminator).

That is, the reaction of complementary strand synthesis is carried out stepwise using four kinds of ddNTP incapable of extension from their 3' ends as reagents for complementary strand synthesis, and genotyping at a target site is performed by bioluminescence. In the method of the present invention, an incorporated nucleotide has no ability of strand extension, and therefore it is unnecessary to add a hydrolytic enzyme such as apyrase. Conventionally, the use of ddATP has been avoided because it generates background luminescence similarly to dATP. However, the present inventors have demonstrated that ddATP has a low activity as a substrate for the luciferase reaction which is different from dATP, and therefore there is no need to use expensive dATPαS. Further, in the present invention, the background bioluminescence is reduced by adding a minute amount of pyrophosphatase (hereinafter, sometimes referred to as PPase) to the reagent mixture, thus allowing genotyping to be performed with high sensitivity. Still further, it is possible to detect multiplex mutation or variation sites in one reaction vessel by the use of a plurality of primers.

According to the present invention, the type of a nucleotide at a specific site and the presence or absence of mutation or variation can be examined by a simple apparatus using a bioluminescent reaction. The nucleotide substrates (ddNTP) used in th present invention have no ability of strand extension from their 3' ends, and when these are incorporated into a DNA strand, complementary strand synthesis does not take place thenafter. Therefore, it is unnecessary to degrade an added nucleotide substrate by using an expensive hydrolytic enzyme such as apyrase. In addition, since ddNTP does not serve as a substrate for luciferase reaction, it is also unnecessary to use an expensive reagent such as dATPαS. The reagents used in the present invention (ddNTP, PPase, etc.) are all inexpensive, and multiplex mutation or variation sites can be detected in one reaction vessel at the same time. Therefore, it is possible to reduce cost and time compared with conventional typing methods.

### BRIEF DESCRIPTION OF THE DRAWINGS

Fig. 1 shows comparison between stepwise reactions of a method of the present invention and stepwise reactions of a pyrosequencing method, where Fig. 1A represents the method of the present invention and Fig. 1B represents the pyrosequencing method;
Fig. 2 shows a reaction scheme in the method of the present invention (Example 1);
Fig. 3 shows comparison between luminescence pattern obtained by the method of the present invention and luminescence pattern obtained by the pyrosequencing method, where Fig. 3A represents the luminescence pattern by the method of the present invention and Fig. 3B represents the luminescence pattern by the pyrosequencing method;
Fig. 4 is a graph showing comparison of luminescent intensities in luciferase reaction when dATP, dATPαS, ddATP, and ddATPαS are used as a substrate, respectively;
Fig. 5 shows a reaction scheme in the method of the present invention in which ATP is synthesized from AMP as a substrate using an enzyme PPDK;
Fig. 6 represents combinations of three SNPs and luminescence patterns observed;
Fig. 7 is a graph showing a luminescence pattern in typing of three SNPs;
Fig. 8 represents graphs showing luminescence patterns in an example of multiplex SNP typing by adding three primers in turn; and
Fig. 9 shows a luminescence pattern obtained when typing is carried out without apyrase (with PPase added at 40 mU/mL) in Example 2.

### DETAILED DESCRIPTION OF THE PREFERRED EMBODIMENTS

The method of the present invention is a method for analyzing a nucleotide sequence by bioluminometric assay coupled with terminator incorporation including a step of synthesizing complementary strand by adding any one of four kinds of ddNTP consisting of ddATP, ddGTP, ddCTP, and ddTTP or their derivatives in a reaction vessel containing a nucleic acid sample to extend one nucleotide at a target site, a step of forming ATP from released pyrophosphate to subject the ATP to a bioluminescent reaction as a reaction substrate, and a step of determining the presence or absence of the complementary strand synthesis by the bioluminescence to type the nucleotide sequence at the target site.

The four kinds of ddNTP or their derivatives are added one by one to a reaction vessel, and the kind of nucleotide at the target site can be determined by luminescence that occurs only when a nucleotide complementary to the detection target site is added. Since ddNTP or their derivatives lack in extension ability from their 3' ends despite the fact that they themselves serve as substrates for complementary strand synthesis, their actions are limited to the complementary strand synthesis and one nucleotide extension, and thus generated bioluminescence always results in a luminescence intensity of one nucleotide. The derivatives of ddNTP include, for example, ddNTPαS such as ddATPαS.

For the bioluminescent reaction using ATP as a reaction substrate, an enzyme-catalyzed luminescent reaction by the luciferin-luciferase system can be employed. The conversion of pyrophosphate to ATP may be carried out by a conventional reaction system that uses APS and ATP sulfurylase. However, the problem of background luminescence can be avoided by using a reaction system in which AMP that does not serve as a substrate for the luciferase reaction and pyruvate phosphate dikinase are included.

When at least an enzyme that degrades pyrophosphate and/or ATP is allowed to be present beforehand in the reaction vessel, pyrophosphate generated by the luciferase reaction and/or ATP is instantly degraded, and therefore the luminescence is converged within a certain time period (about one min) and a distinct peak is obtained. For the enzymes that degrade pyrophosphate and ATP, pyrophosphatase and apyrase can be used respectively, and pyrophosphatase is preferred in view of low cost and high activity.

The method of the present invention can be utilized for detection of all cases of single nucleotide mutations and variations including one nucleotide substitutions (SNPs), nucleotide mutations and methylations (methylcytosine, etc) associated with malignant transformation, artificial nucleotide substitutions, and the like. The detectable site is not limited to one, and two or more target sites can be readily typed in one reaction vessel as long as the conditions described later are met.

In typing of multiplex target sites, primers corresponding to each site are prepared, and complementary strand synthesis and bioluminescent reaction are carried out using two or three primers of the prepared primers at the same time as well as the above four kinds of ddNTP or their derivatives one by one. From the results obtained, the target sites corresponding to the above two or three primers are genotyped, respectively. Further, complementary strand synthesis and bioluminescent reaction are carried out using one primer and the above four kinds of ddNTP or their derivatives one by one, and the target site corresponding to the primer is determined. Subsequently, a ddNTP or derivative thereof added to the reaction vessel is degraded or removed, and then a next target site may be determined with its corresponding primer, which may be continued by turns. Still further, the process of detecting two or more target sites simultaneously by adding two or more primers to the reaction vessel at the same time may be repeated. It should be noted that the method for degrading or removing ddNTP includes the enzymic degradation described above and a method of washing out with the reaction solution by immobilizing a nucleic acid sample.

The present invention also provides a reagent kit for use in the above method for analyzing a nucleotide sequence. This kit includes
1) four kinds of ddNTP corresponding to A, G, T, and C, or their derivatives
2) DNA polymerase
3) luciferin and luciferase
4) AMP, phosphoenol pyruvate (PEP), and pyruvate phosphate dikinase, and/or APS and ATP sulfurylase.
   Further at least an enzyme that degrades pyrophosphate and/or ATP may be added to the kit as required.

### EXAMPLES

Hereinafter, the present invention will be explained in more detail by means of examples, but is not limited to these examples.

### Example 1

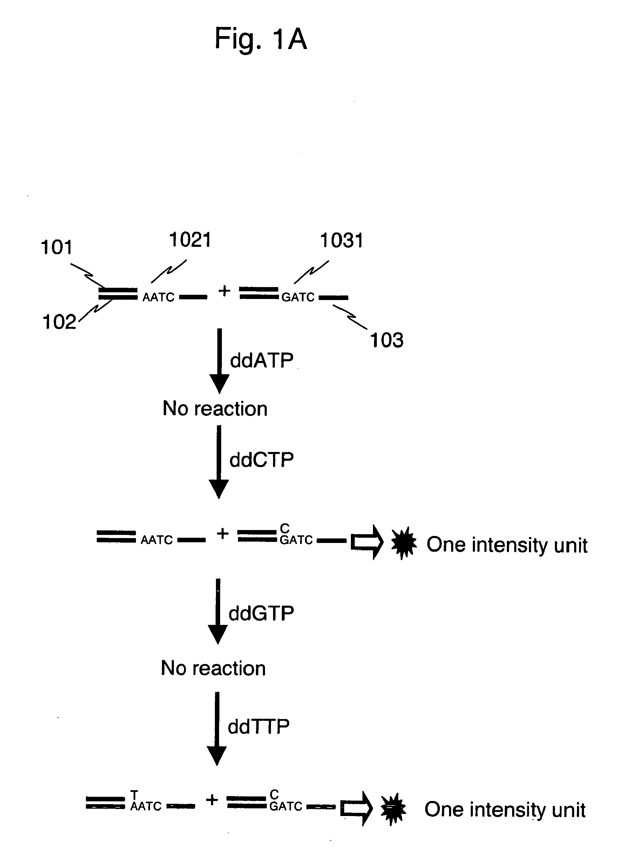

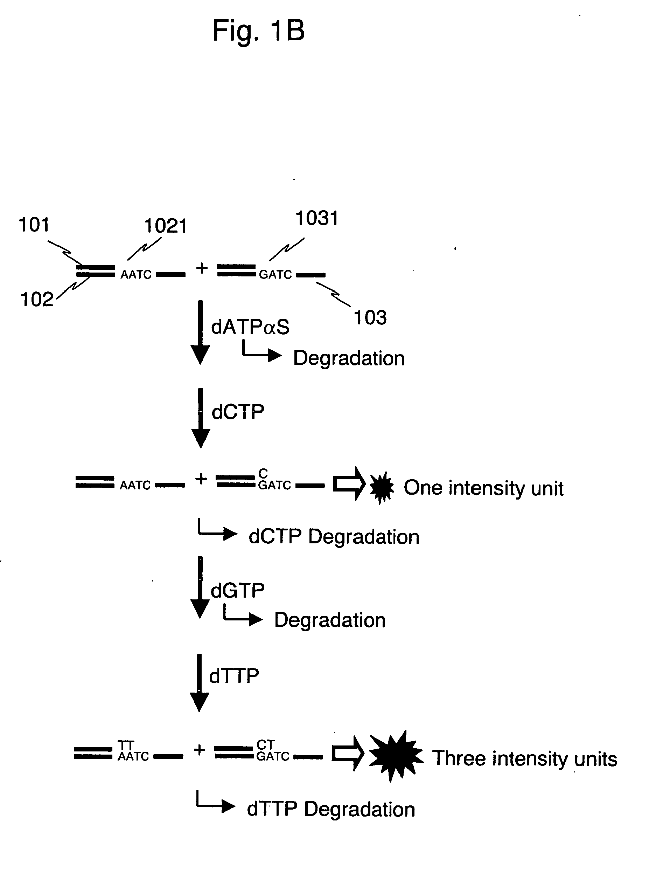

Fig. 1 shows comparison between stepwise reactions according to a method of the present invention and stepwise reactions of a pyrosequencing method. The gene of a human individual consists of a pair of two kinds of alleles derived from father and mother, respectively. In the present example, an example of a heterozygous sample having each of two kinds of alleles different in A/G at a polymorphism site is explained.

Each of the numerals 102 and 103 in Fig. 1 shows one strand of DNA from each of two alleles representing a polymorphism A (1021) and a polymorphism G (1031), respectively. With the use of a common primer 101, SNP typing according to the method of the present invention is shown in Fig. 1A, and SNP typing by a conventional pyrosequencing method is shown in Fig. 1B. Luminescence intensity when a reaction of complementary strand synthesis takes place is proportional to the number of nucleotides incorporated in the complementary strand synthesis. For simplicity, the following will be explained assuming that the luminescence intensity corresponding to one nucleotide is "one intensity unit (or one unit)". Since ddNTP is used as a substrate in the method of the present invention, the number of nucleotide to be extended is always one, and the resulting luminescence intensity is one intensity unit. On the other hand, since dNTP is used as a substrate in the pyrosequencing method, the nucleotide length to be synthesized differs depending on the kind or condition of a nucleotide adjacent to the polymorphism site of the typing target, and therefore the luminescence intensity results in various values such as one unit, two units, and three or more units.

A protocol of the typing was as follows. First, a target DNA was amplified. After converting it to the single strand DNA, it was placed in a reaction vessel. To this single strand DNA was hybridized a primer designed such that its 3' end corresponded to one nucleotide upstream of the target nucleotide. In other words, a nucleotide substrate was designed to be incorporated into the typing target site in the first complementary strand synthesis from the primer. In this example, the nucleotide to be incorporated into the typing target site was T or C. Four kinds of nucleotide substrates, i.e. nucleotide analogs (terminators) or nucleotides, were added into the reaction vessel via a dispenser by turns. The kinds and the order of the nucleotide substrates to be added were ddATP→ddCTP→ddGTP→ddTTP in the method of the present invention, while those were dATPαS→dCTP→dGTP→dTTP in the pyrosequencing method. When the added nucleotide was complementary to the nucleotide at the target site, the reaction of complementary strand synthesis proceeded and the added nucleotide bound to the 3' terminal side of the primer. In the present example, an apyrase was added to the reaction solution beforehand because of its necessity in the pyrosequencing, and therefore added nucleotide substrates were degraded within one minute. However, this hydrolytic degradation by apyrase was not necessarily required in the method of the present invention as described later.

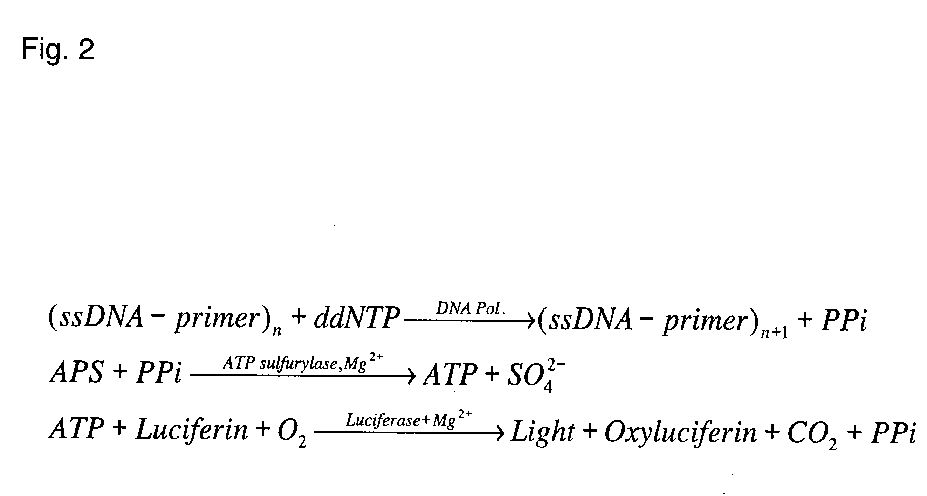

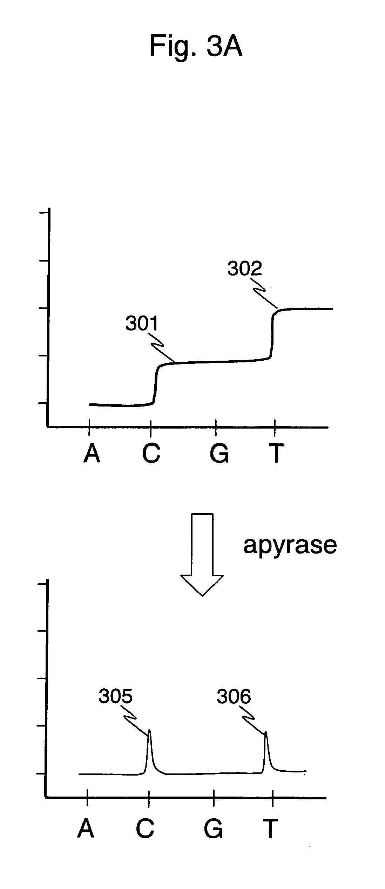

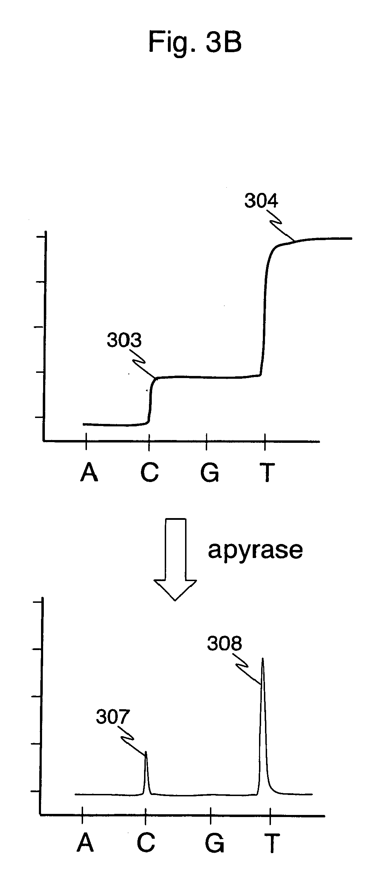

Since ddATP or dATPαS that were added first were not complementary to the nucleotide of the polymorphism site that was the typing target, no reaction of complementary strand synthesis took place. Next, when ddCTP or dCTP was added, a reaction of complementary strand synthesis took place only at the allelic DNA strand 103 in which the polymorphism site was G, and luminescence of one intensity unit was observed in both of the method of the present invention and the pyrosequencing method. Similarly, since a further next ddGTP or dGTP was not complementary to the nucleotide of the polymorphism site that was the typing target, no reaction of complementary strand synthesis took place, and no luminescence was observed. Still further next, when ddTTP or dTTP was added, luminescence of one intensity unit was observed in the method of the present invention due to a reaction of complementary strand synthesis at the allelic DNA strand 102 in which the polymorphism site was A. On the other hand, dTTP was incorporated into both of the polymorphism site in the DNA chain 102 and its adjacent site, and in addition, one dTTP was incorporated into the other allelic DNA strand 103, thereby resulting in observing luminescence of a total of three nucleotides (three intensity units). A sequential reaction scheme in the method of the present invention is shown in Fig. 2. The luminescence (signal intensity) observed in the method of the present invention (A) and that of the pyrosequencing method (B) are schematically shown in Fig. 3.

In the method of the present invention, added nucleotides are incorporated into only mutation or variation sites depending on the existing nucleotides of interest. Therefore, when a DNA sample is derived from a subject with homozygous genotype, luminescence of two units appears at a location. When a DNA sample is derived from a subject with heterozygous genotype, luminescence of one unit appears at two locations. On the other hand, appearance of the luminescence intensity in pyrosequencing results in a complex pattern because of continuation of complementary strand synthesis.

The composition of the reaction solution used in the present example is shown in Table I. The reaction solution contained a DNA polymerase, ATP sulfurylase and APS used for ATP formation reaction, and luciferase and luciferin used for luminescent reaction. In the pyrosequencing method, added nucleotide substrates had to be decomposed, and therefore a degradation enzyme, apyrase, was added to the reaction solution. As described later, the reagents often contain pyrophosphate and the like as impurities, and therefore a minute amount of pyrophosphatase (PPase) was added beforehand in a reagent containing a nucleotide analog in the method of the present invention.

**Table I Composition of reaction solution**

| Reagent | Concentration |
|---|---|
| Tricine (pH 7.8) | 60 mM |
| EDTA | 2 mM |
| MgAc | 20 mM |
| DTT | 0.2 mM |
| ATP sulfurylase | 0.2 U/mL |
| Luciferase | 50.0 GLU/mL |
| Sequenase 2.0* | 65 U/mL |
| Apyrase | 1 U/mL |
| Luciferin | 0.4 mM |
| APS | 5 µM |
| BSA | 0.1 % |

| | |
|---|---|
| * Thermostable DNA polymerase for cycle sequencing (Amersham Biosciences Ltd.) | |

When a nucleotide substrate complementary to a template DNA is added, complementary strand synthesis takes place and luminescence is observed. However, when the complementary strand synthesis takes place, pyrophosphate is released as a byproduct. The pyrophosphate forms ATP in the presence of APS and ATP sulfurylase, and APS itself serves as a substrate for the luciferase reaction, thereby causing a luminescent reaction. Since multi-step complementary strand extension reactions take place in pyrosequencing, it is necessary to add a sufficient amount of APS in advance, which leads to a high background luminescence. In the method of the present invention, it was unnecessary to add such a large amount of APS as the case in pyrosequencing, and thus the background luminescence due to APS was practically negligible.

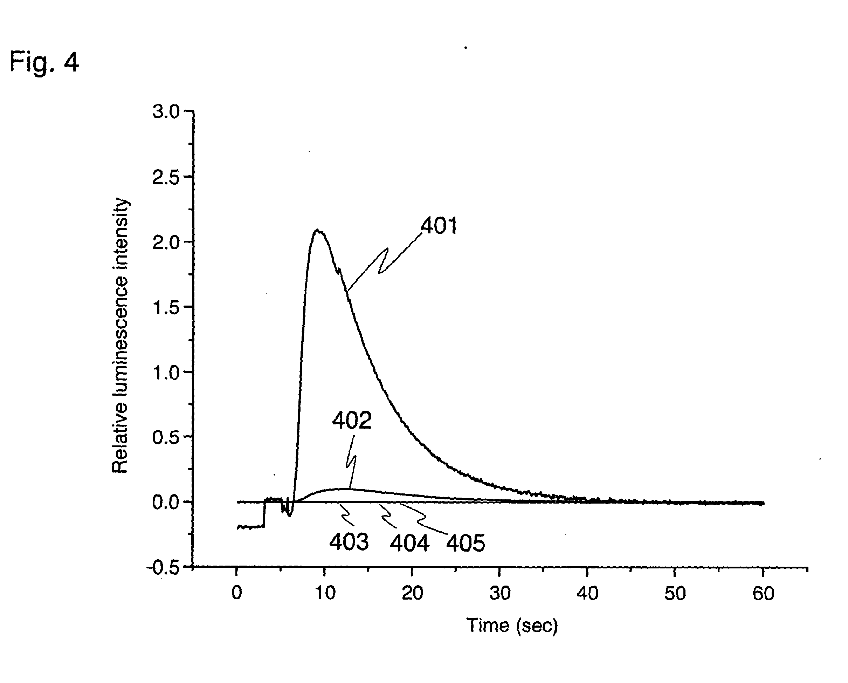

ATP reacts with luciferin in the presence of luciferase to emit light and release pyrophosphate. The pyrophosphate reacts again with APS to form ATP, and therefore the luminescent reaction continues until luciferin is exhausted or APS is consumed. However, when apyrase is added, the apyrase also decomposes ATP and luminescence decays in a relatively short time of approximately one minute. The luminescence (signal intensity) observed in this way in the presence of apyrase is shown in Fig. 4. In the present example, the amount of PPase was kept low. Therefore, when complementary strand synthesis took place, signal intensity rapidly rose and then slowly decayed in accordance with a mild degradation reaction of pyrophosphate (401). Subsequently when a next nucleotide analog was added and incorporated by complementary strand synthesis, the signal intensity rapidly rose again, thus allowing easy genotype detection.

As described above, various reagents used for the reaction often contain pyrophosphate (hereinafter, sometimes referred to as PPi) as an impurity. The pyrophosphate is converted to ATP when the reagents for the reaction are mixed and gives rise to a luminescent reaction, which causes a background luminescence. For this reason, a minute amount of PPase was allowed to be present in the reagent containing a nucleotide analog, thereby degrading pyrophosphate that was an impurity. When the amount of PPase added was increased, the amount of pyrophosphate in the reaction cycle of ATP→PPi→ATP decreased, and thus the luminescence decayed in a short time. Although the process in which pyrophosphate released by complementary strand synthesis was converted to ATP was also influenced and luminescence (signal intensity) became smaller, there was no disadvantage of measurement.

In place of PPase described above, apyrase can also be used. Since apyrase degrades ATP, dNTP, ddNTP, and the like, not only is luminescence converged in a short time by degrading ATP as described above but also the added nucleotide analogs (ddNTP) are degraded. However, PPase is available at about half price of apyrase, and the amount of PPase necessary for use is about 1/10 of that of apyrase; therefore, cost reduction becomes possible by the use of PPase.

In the present example, terminators, i.e. ddATP, ddCTP, ddGTP, and ddTTP, were used as nucleotide analogs. Luminescent intensities in the luciferase reaction when dATP, dATPαS, ddATP, and ddATPαS were used as a substrate, respectively, are shown in Fig. 4 (401: ATP, 402: dATP, 403: dATPαS, 404: ddATP, 405: ddATPαS). The activity of dATP as a reaction substrate for the luciferase reaction was about 300-fold higher than that of dATPαS, while ddATP exhibited only an activity that is almost as low as dATPαS. ddATP is available at about half price of dATPαS, and further the amount of ddATP necessary for the reaction is about 1/10 of that of dATPαS; therefore, reduction in typing cost becomes possible by using the method of the present invention with ddATP compared with a conventional method.

Since one unit of luminescence was observed in the method of the present invention only when a nucleotide was incorporated into the 3' end of a primer, the obtained data (luminescence pattern) was simple and obvious. The target single nucleotide polymorphisms (SNPs) were readily discernible by the luminescence generated only when a nucleotide complementary to the detection target site was added. According to the method of the present invention, SNPs can be easily typed with the use of inexpensive reagents, ddATP and PPase, as described above.

### Example 2

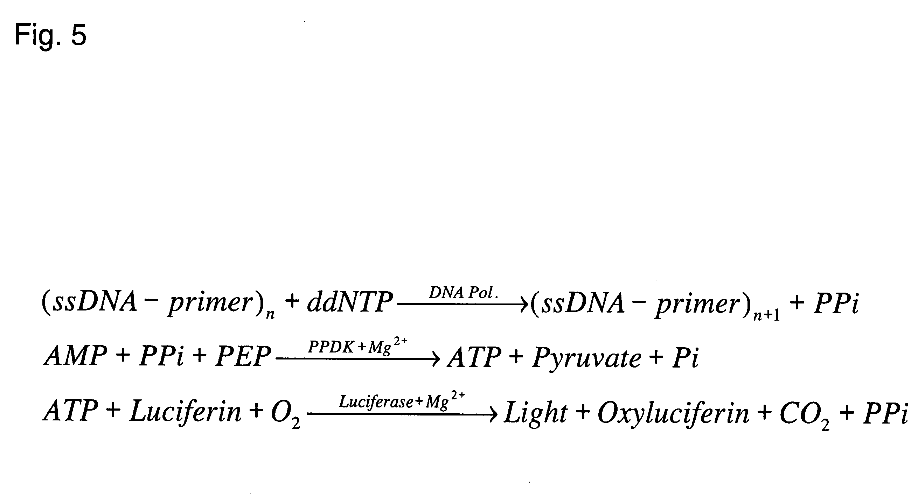

In the example 1, the reaction for the conversion of pyrophosphate to ATP made use of ATP sulfurylase by which ATP was formed from pyrophosphate and APS. Although the activity of APS as a luminescent substrate for luciferase is low as mentioned above, luminescence caused by APS became nonnegligible when APS was present in a large amount in the reaction vessel. Moreover it sometimes lowered the sensitivity of detection. For this reason, a system in which AMP that is unable to serve as a substrate for luciferase reaction is used as a substrate for reaction with pyrophosphate and both are reacted with the use of pyruvate phosphate dikinase (PPDK) will be explained. The outline of the reaction in the present example is shown in Fig. 5. Although the method of the present example was almost the same as that of the example 1 except for ATP formation process, reagents and reaction conditions to be used differed to some extent because conditions were set to become appropriate for the enzyme used, PPDK. The composition of the reaction solution used in the present example is shown in Table II.

**Table II Composition of reaction solution**

| Reagent | Concentration |
|---|---|
| Tricine (pH 7.8) | 60 mM |
| EDTA | 2 mM |
| MgAc | 20 mM |
| DTT | 0.2 mM |
| PPDK | 10.0 U/mL |
| Luciferase | 200.0 GLU/mL |
| Sequenase 2.0* | 65 U/mL |
| Apyrase | 1 U/mL |
| Luciferin | 0.4 mM |
| PEP·3Na | 0.04 mM |
| AMP | 0.2 mM |
| BSA | 0.1% |

| | |
|---|---|
| * Thermostable DNA polymerase for cycle sequencing (Amersham Biosciences Ltd.) | |

Since the background luminescence in the reaction system that made use of PPDK was low, it was possible to increase the amount of luciferase and enhance luminescence intensity severalfold. Accordingly, it became possible to measure even a minute quantity of sample with excellent sensitivity.

A protocol of the typing was as follows. Four kinds of ddNTP were added in a reaction vessel by turns to carry out reactions of complementary strand synthesis. The reaction of complementary strand synthesis took place only when a nucleotide analog complementary to the nucleotide at the typing target site was added, and formed pyrophosphate (PPi). The PPi reacted with AMP in the presence of PPDK to form ATP. ATP served as the substrate for the luciferase reaction to generate luminescence, and at the same time formed AMP and PPi. Similarly to the example 1, the formed PPi reacted with AMP to form ATP again. PPi was degraded with PPase or apyrase in the present example as well, and therefore the luminescence intensity decreased gradually. The genotype of a typing target sample was either homozygous or heterozygous. When the sample was homozygous, a rapid increase of luminescence intensity associated with complementary strand synthesis was observed once. When the sample was heterozygous, it was observed twice. It was possible to determine variation at the typing target site by observing such changes in luminescence intensity.

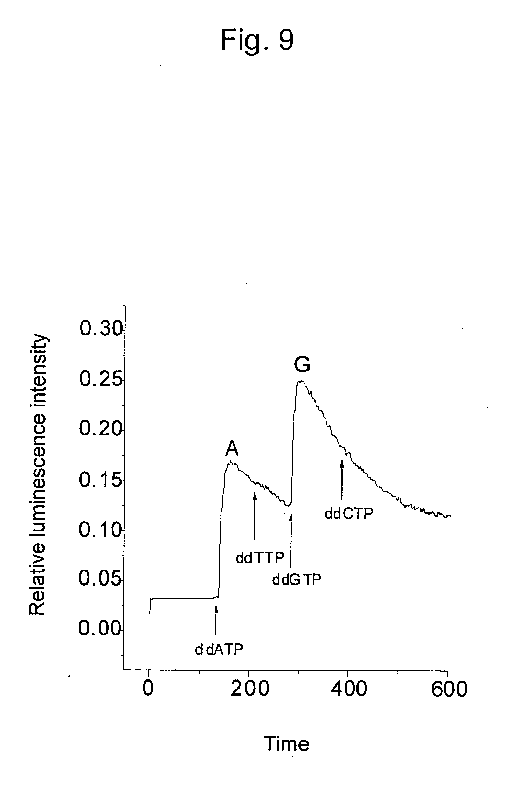

Fig. 9 represents an example in which apyrase was omitted from the reagents in Table II. The polymorphism was A/G heterozygote. It was apparent that luminescence intensities increased rapidly only when nucleotides corresponding to the polymorphism were added. When four kinds of ddNTP or their derivatives were reacted by turns, rapid increase of luminescence intensity was observed once in the case of homozygote, while it was observed twice in the case of heterozygote. In the present example, apyrase was not added and PPase was added instead at a concentration of 40 mU/mL. Therefore, PPi formed by extension was degraded by PPase; and luminescence intensity decayed gradually. The decay was more gradual compared to when apyrase was added. Since ddNTP and their derivatives were used in the present invention, the number of nucleotides that could be extended was always one and its determination was simple. Therefore, it was possible to determine the difference in nucleotides without adding apyrase as a reagent as demonstrated in this example.

### Example 3

The method of the present invention allows multiplex SNPs to be typed in one reaction vessel. As a first example, a case in which typing targets are three SNPs will be explained.

In most cases, SNPs consist of a general type (wild type) and a type in which the one nucleotide of concern is substituted by a specific nucleotide of the other three nucleotides (mutant type). In other words, variations at a SNP site are usually two kinds. Therefore, when three SNPs are typed at the same time, the number of nucleotides to be identified is six in total (2x3). Since the nucleotides present in nature are four kinds consisting of A, C, G, and T, possible combinations of the six nucleotides are 27 in total assuming that SNPs with an identical combination are not measured for the three SNPs at the same time. Thus, the conditions required for complete identification of three SNPs are as follows.

First, it is necessary to select typing target SNPs such that three SNPs do not have a combination identical to each other. Further, it is essential that at least one nucleotide from each of the four nucleotides ACGT is present in the nucleotides of the three SNPs, that is, in the six nucleotides. A case in which two identical combinations are contained in the three kinds of SNPs as exemplified by "A/G" in (A/G, A/G, T/C) should be excluded, and it is necessary that all of the four nucleotides ACGT are contained in the three kinds of SNPs; an example to be excluded is exemplified by (A/G, A/C, G/C) in which "T" is not contained.

These limiting conditions required for combinations of typing target SNPs can simply be set by combinations of their corresponding primers, and therefore the generality of this method is not impaired. The method of multiplex SNP typing according to the present invention will be specifically explained below by way of an example in which three kinds of typing target SNPs, SNP-1, SNP-2, and SNP-3, are A/C, A/G, and C/T, respectively.

First, a reaction solution was prepared by adding three kinds of primers to a target DNA. To this solution were added four kinds of ddNTP by turns, followed by observation of luminescence intensities as explained in the examples 1 and 2. When the added nucleotide was not incorporated, the luminescence intensity was zero, and when the nucleotide was incorporated at one typing target site, the luminescence intensity was one. Further, when the added nucleotide corresponded to one homozygous SNP genotype or two heterozygous SNP genotypes, the luminescence intensity became two in either case. Furthermore, as to the luminescence intensities obtained when nucleotide analogs other than ddATP were added, the luminescence intensities were two when the corresponding SNP alleles were homozygous and one when the corresponding SNP alleles were heterozygous, though the luminescence intensity was zero when a SNP site was A and homozygous for the alleles. In this way, all polymorphism sites could be determined by comparing the luminescence intensities for the added nucleotides.

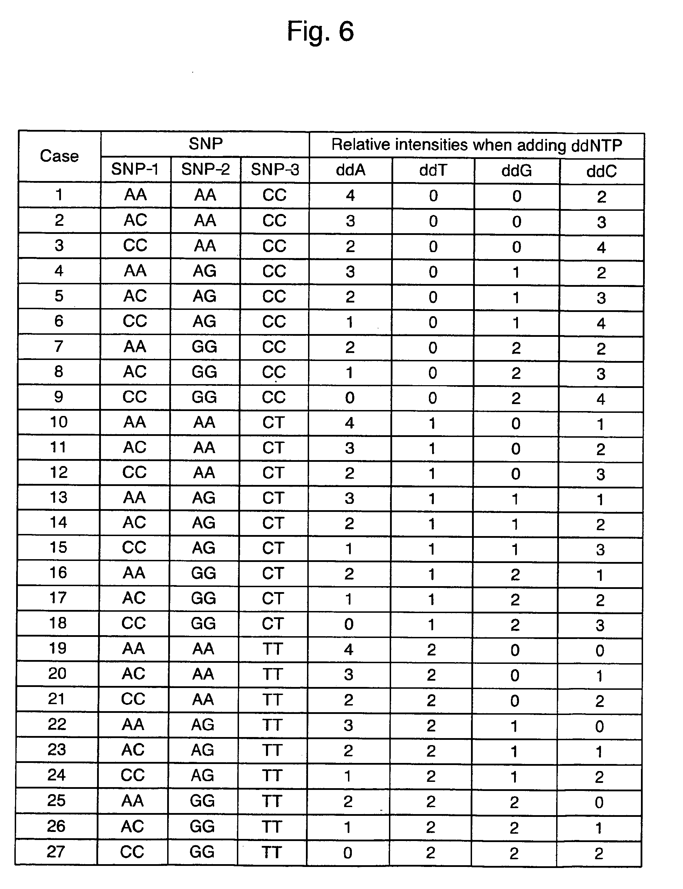

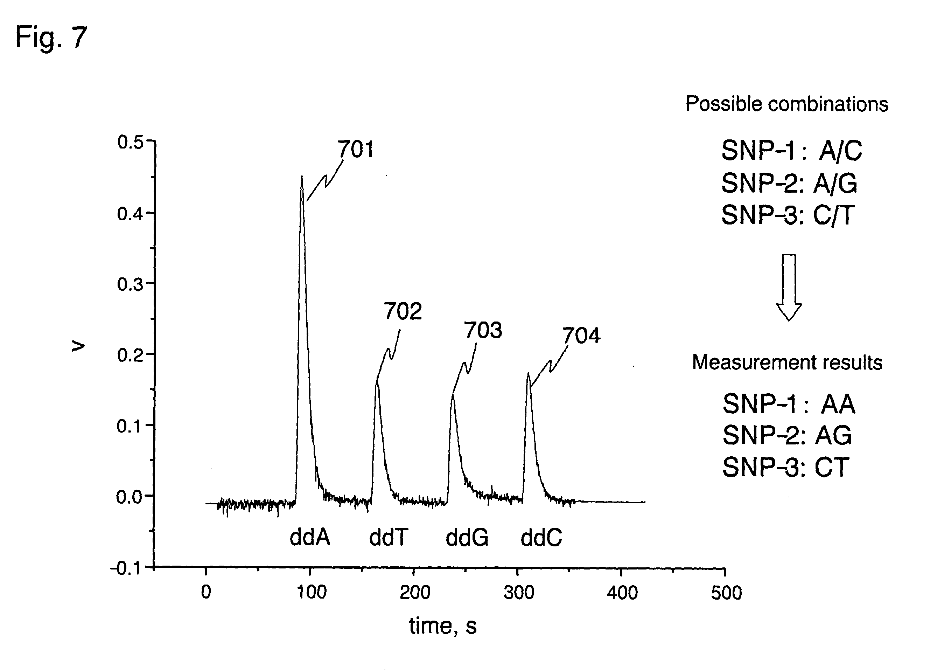

Fig. 6 shows what kinds of luminescence patterns are observed depending on various combinations of the three kinds of typing target SNPs. The total of luminescence intensity is six units in any combinations. The luminescence patterns of these 18 combinations are all different, and it is found that polymorphism sites can be uniquely identified from the obtained luminescence pattern. Fig. 7 shows an example of practical measurement. In this example, a sample having three SNPs of A/C, A/G, and C/T was used. Since the luminescence intensity was one unit when T, G, and C were added respectively (702, 703, and 704) and three units when A was added (701) in the measurement example, the genotype of the sample was identified as (AA, AG, CT). This corresponded to Case 13 in Fig. 6.

As is apparent from Fig. 6, any SNPs up to three can be uniquely identified according to the method of the present invention as long as the above-described conditions are met. In addition, the method can be similarly applied to cases where the target SNPs are present on one DNA strand and on different DNA strands.

In the method of the present invention, addition of ddNTP was once for each nucleotide, and even if added ddNTP remained, there was no harm in measurement. Therefore, it was unnecessary to add any degradation enzyme besides PPase contained beforehand in the reagent. Although apyrase was added to the reaction solution in order to observe luminescent signals in sharp peak in the present example, it is of course possible to measure without its addition.

### Example 4

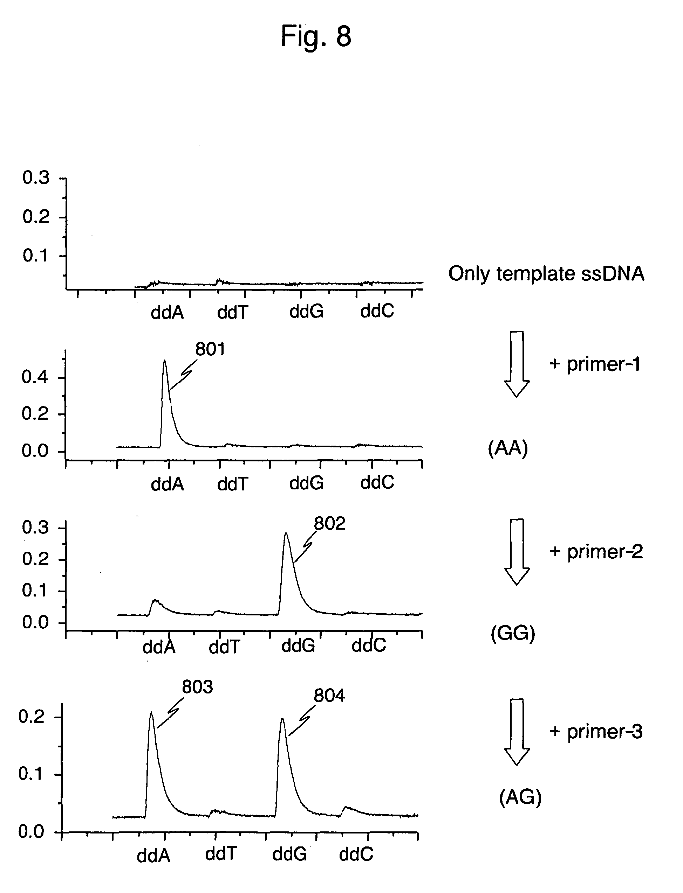

Next, an example of multiplex SNP typing will be explained. In the case of multiplex SNP typing, a plurality of primers were added to the reaction vessel by turns, and further each of the four kinds of nucleotide analogs was added therein by turns and measured (Fig. 8). The reaction temperature was raised to near 40 degrees C (desirably, 32 to 40 degrees C) and the addition amounts of primers were as low as possible so that primers might not partially hybridize to each other and inhibit the reaction of complementary strand synthesis. Preferably, the amount of each primer to be added was approximately equimolar to a target DNA sample.

Luminescence was not generated when ddNTP was added in the absence of a primer in the reaction vessel (the uppermost graph in Fig. 8). In typing of multiplex SNP sites, added ddNTP had to be removed because it interfered with the measurement using the next primer. For the removal of ddNTP, there are a method of degrading it with a hydrolytic enzyme such as PPase or apyrase and a method of washing it out with the reaction solution.

In a first measurement with the use of a first primer (primer-1), luminescence (signal) was observed only when ddATP was added (801). This indicated that the site corresponding to the added primer was homozygote of genotype (AA). In a second measurement in which a new primer was added, apyrase was added to the reaction vessel to degrade the remaining ddNTP used previously because its presence was disadvantageous. Since the ddNTP might be degraded by the time of a cycle when the same nucleotide was added in the measurement with the use of a subsequent primer, the addition of less amount of apyrase was sufficient compared to pyrosequencing.

In a measurement with the use of a second primer (primer-2), weak luminescence (signal) due to ddATP was observed. It was considered from its intensity that ddATP was incorporated into the site unreacted in the first measurement. In the second measurement, intense luminescence (signal) was observed when ddGTP was added (802). This indicated that the site corresponding to the second primer was homozygote of genotype (GG).

In a measurement with the use of a third primer (primer-3), luminescence having approximately equal intensities was obtained when ddATP and ddGTP were added (803). This indicated that the site corresponding to the third primer was heterozygote of genotype (AG).

In this way, genotypes of each of multiplex SNPs can be determined by adding different primers by turns and carrying out measurement every time. In the present example, primers were added one by one in order, though it is possible to add three at a time. In this case, the addition of primers three or four times allows a total of ten or more SNPs to be determined.

The method for analyzing a nucleotide sequence of the present invention can be effectively exploited in genotyping of specific sites, particularly genotyping of mutations and variations including single nucleotide polymorphisms (SNPs) and methylations.

## Claims

1. A method for analyzing a nucleotide sequence comprising the steps of:
carrying out complementary strand synthesis by adding at least one of four kinds of ddNTP corresponding to nucleotides A, G, T, and C, or derivatives thereof to a reaction vessel containing a nucleic acid sample to extend one nucleotide at a target site;
performing a bioluminescent reaction with the use of ATP formed from released pyrophosphate as a reaction substrate; and
typing the target site by determining the presence or absence of the complementary strand synthesis based on a result of the bioluminescent reaction.

2. The method for analyzing a nucleotide sequence according to claim 1, wherein the four kinds of ddNTP or the derivatives thereof are added to the reaction vessel one by one to perform complementary strand synthesis.

3. The method for analyzing a nucleotide sequence according to claim 1, wherein ATP is formed from pyrophosphate released by the complementary strand synthesis with the use of pyruvate phosphate dikinase and AMP.

4. The method for analyzing a nucleotide sequence according to claim 1, wherein the bioluminescent reaction is a luminescent reaction catalyzed by luciferase.

5. The method for analyzing a nucleotide sequence according to claim 1, wherein at least an enzyme capable of degrading any one of pyrophosphate and ATP or both is allowed to be present in the reaction vessel.

6. The method for analyzing a nucleotide sequence according to claim 5, wherein the bioluminescence is converged within a certain period of time by allowing any one of pyrophosphate generated from the bioluminescent reaction and ATP or both to be degraded by the enzyme.

7. The method for analyzing a nucleotide sequence according to claim 5, wherein the enzyme is any one of pyrophosphatase and apyrase or both.

8. A method for analyzing nucleotide sequences at least two target sites with the use of the method according to claim 1.

9. The method for analyzing nucleotide sequences at least two target sites according to claim 8, comprising:
preparing primers corresponding to each of the target sites;
performing complementary strand synthesis and bioluminescent reaction with the use of two or three primers thereof at the same time and the four kinds of ddNTP or the derivatives thereof one by one; and
typing the target sites corresponding to the two or three primers based on results of the bioluminescent reaction.

10. The method for analyzing nucleotide sequences at least two target sites according to claim 8, comprising:
preparing primers corresponding to each of the target sites;
performing complementary strand synthesis and bioluminescent reaction with the use of one primer at a time and the four kinds of ddNTP or the derivatives thereof one by one;
typing the corresponding target site;
degrading or removing ddNTP or the derivative thereof; and
typing a next target site with a next primer by turns.

11. The method for analyzing nucleotide sequences at least two target sites according to claim 8, comprising:
preparing primers corresponding to each of the target sites;
performing complementary strand synthesis and bioluminescent reaction with the use of at least two primers thereof at the same time and the four kinds of ddNTP or the derivatives thereof one by one;
typing the corresponding target sites;
degrading or removing ddNTP or the derivatives thereof; and
typing other target sites with one, or two or more primers repeatedly in turn.

12. The method for analyzing a nucleotide sequence according to claim 1, wherein the target site is a single nucleotide mutation or variation site including SNP.

13. A kit for analyzing a nucleotide sequence comprising the following 1) to 4):
1) four kinds of ddNTP corresponding to A, G, T, and C, or derivatives thereof;
2) DNA polymerase;
3) luciferin and luciferase; and
4) a combination of AMP, phosphoenol pyruvate (PEP), and pyruvate phosphate dikinase or a combination of APS and ATP sulfurylase.

14. The kit for analyzing a nucleotide sequence according to claim 13, wherein at least an enzyme capable of degrading any one of pyrophosphate and ATP or both is further included.
